# EUROPEAN PATENT APPLICATION

(11) **EP 1 721 894 A1**
(43) Date of publication of application: **15.11.2006**
(21) Application number: 06425309.9
(22) Date of filing: 08.05.2006
(51) Int. Cl.: C07C 405/00

(54) **Process for the synthesis of prostaglandin derivatives**

(30) Priority: 13.05.2005 IT RM20050231
(71) Applicant: Technopharma S.A., 6900 Lugano, Svizzera (IT)
(72) Inventor: Lisi, Giuseppe, 6900 Lugano (CH)
(74) Representative: Banchetti, Marina

(57) **Abstract**

An alternative method for the synthesis of prostaglandin F analogues, in particular, analogues of PGF_{2α} and specifically for the synthesis of latano-prost, wherein the transformation of the lactone intermediate (5) 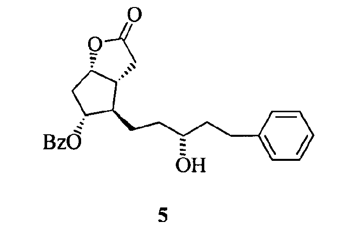 into the corresponding lactol (7) 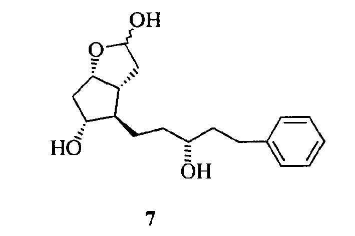 is carried out by treating the lactone intermediate (5) with a silane, preferably polymethylhydrosiloxane (PMHS), in the presence of a titanocene - and preferably titanocene fluoride.

The method enables considerable production economies with respect to the conventional reduction of lactone with diisobutylaluminum hydride (DIBAL-H).

## Description

The present invention concerns a process for the synthesis of prostaglandin F derivatives. More specifically, the invention concerns a new method for the synthesis of prostaglandin analogues of the F_{2α} type (PGF_{2α}), including, in particular, latanoprost, which is a pharmaceutical active ingredient of considerable interest in the ophthalmic field. The method is essentially based on the introduction of a specific innovative step in the general scheme of prostaglandin synthesis of the latanoprost type, thanks to which it is possible to achieve considerable production economies both in terms of yield and in terms of simplicity of the operative conditions and availability of the starting ingredients.

As is known, prostaglandins are a family of biologically active substances ubiquitously produced in the body. They have extremely complex biological functions, among which stimulating the smooth muscles, dilating the smaller arteries and the bronchi, lowering blood pressure, inhibiting gastric secretion, and promoting platelet aggregation. Their general structure may be traced back to a hypothetic base structure, prostanoic acid - a fatty acid of twenty carbon atoms characterised by a 5-member ring bound to two linear lateral chains of seven and eight carbon atoms, of which the seven-atom chain (α chain) has a carboxy terminal group. The various prostaglandin (PG) subclasses, which differ from one another for the specific characteristics of their five-member ring, are identified by a capital letter of the Latin alphabet (from A to I), as shown below as an example.

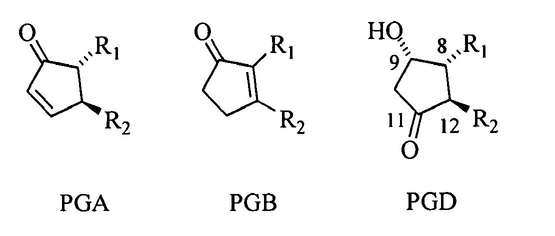

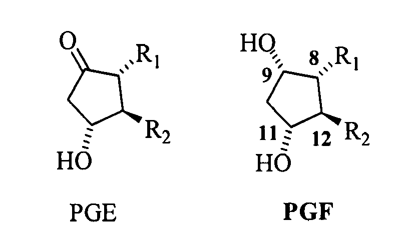

The saturation degree, in terms of double bonds present in the two lateral chains, is indicated by an Arabic numeral as subscript to the subclass name, according to the following scheme:

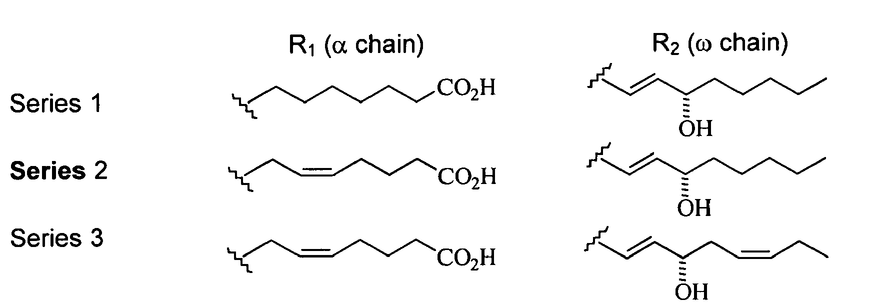

Finally, the Greek letters α and β are used to indicate the configuration in C-9: α indicates that the substituent is below the plane (as in natural prostaglandins), while β indicates that it is above the plane (as in some synthetic analogues).

The F_{2α} type prostaglandin (PGF_{2α}, 7-[3,5-dihydroxy-2-(3-hydroxy-1-octenyl)cyclopentyl]-5-heptenoic acid) and its corresponding esters, in particular the isopropyl ester, have long been known for their capacity to favour aqueous humour flow, thereby significantly reducing intraocular pressure. Although these compounds are active in the treatment of glaucoma and ocular hypertension, a limiting factor of their use in ophthalmic therapy is their tendency to cause surface irritation, blood vessel dilation in the conjunctiva and probably also irritating effects to the cornea.

Particularly to avoid these side effects, the international patent application WO 90/02553 (Pharmacia AB) proposes some prostaglandin derivatives (A, B, D, E or F) wherein the ω chain has been modified by inserting an aromatic ring. In particular, the phenyl-substituted analogues of PGF_{2α}, possibly having a saturated ω lateral chain, resulted to be therapeutically more selective than PGF_{2α} and its corresponding esters. Among these there is also 13,14-dihydro-17-phenyl-18,19,20-trinor-PGF_{2α}, an active ingredient that later became known internationally as latanoprost.

Other examples of PGF_{2α} analogues of therapeutic use as antiglaucoma agents are cloprostenol, which contains a chlorophenyl-ether end substituent on the ω chain, fluprostenol, containing a trifluoromethyl-phenyl ether substituent in the same position, travoprost, corresponding to the isopropyl ester of the previous compound, bimatoprost (17-phenyl-18,19,20-trinor-PGF_{2α} N-ethyl amide), containing a phenyl substituent on the ω chain and having the carboxy end group of the α chain modified with an amide function.

For total prostaglandin synthesis the stereochemistry of the various carbon atoms must be taken into account; in particular, the problem concerns the introduction of the chirality required at C-8, C-9, C-11, C-12 and C-15. In 1969 Corey and co-workers (Corey et al., J. Am. Chem. Soc. 91, (1969), 5675; Corey et al., J. Am. Chem. Soc. 92, (1970), 2586; Corey et al., Tetrahedron Letters 307, (1970)) described a stereocontrolled synthesis of prostaglandins of the PGE₂ and PGF_{2α} type. The basic idea of the synthesis method developed by Corey envisaged the synthesis of an intermediate **(I)** 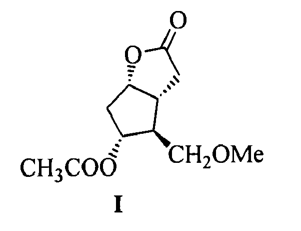 in which the stereochemistry required at C-8, C-9, C-11 and C-12 was already well established. This compound, synthesised through five reaction stages by Corey himself, is known as the Corey's lactone, and is commercially available. The intermediate **I** is currently the starting point for synthesising various prostaglandins, according to a general scheme envisaging in a first phase the introduction of the desired ω chain on the cyclopentane unit and in a second phase the introduction of the α chain, which normally passes through the transformation of the lactone ring into a cyclic hemiacetal function (lactol), which is then condensed with the group desired for the α chain by a Wittig reaction.

The aforesaid basic document WO 90/02553, dedicated to the proposition of the broad family of prostaglandin derivatives defined therein as antiglaucoma agents, having a better efficacy and greater tolerability than the previously known prostaglandins, reports - for the synthesis of the compounds studied - partly methods limiting themselves to suitably functionalising the commercially available prostaglandins and partly syntheses that essentially follow Corey's scheme, starting from the lactone of formula I, or rather, from the corresponding alcohol.

International patent application WO 93/00329 (Kabi Pharmacia AB and Chinoin) is considered to be the reference patent document for the synthesis of esters of 13,14-dihydro-17-phenyl-18,19,20-trinor-PGF_{2α}, and thus for the synthesis of latanoprost. The process described focuses on the synthesis process steps that follow the introduction of the ω chain. In the same period, one of the authors of the above patent published a scientific work (Resul, B., et. al., *J*. *Med. Chem.* **36** (1993), 243) summarising the synthesis process proposed for the preparation of latanoprost. This sequence is reported in the following scheme.

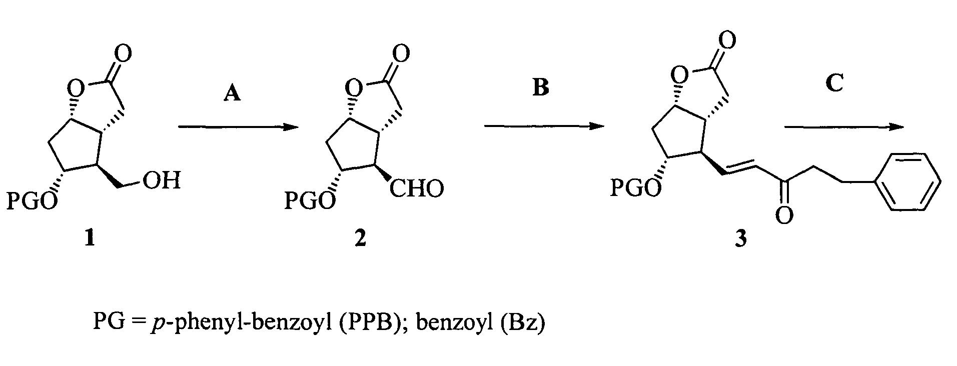

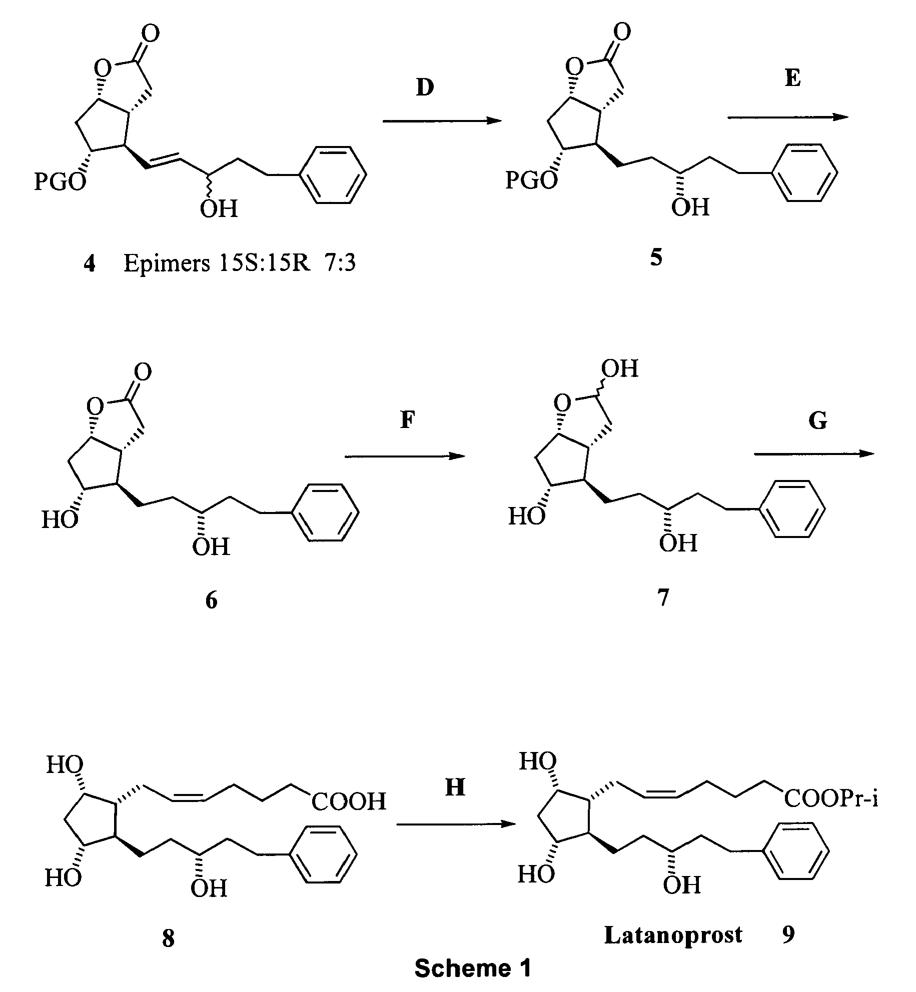

The starting material is still the bicyclic lactone (**1**) protected at the secondary hydroxy group with the *p*-phenyl-benzoyl (PPB) group or with the benzoyl (Bz) group. At this reaction stage, the primary alcohol group of the Corey lactone is oxidised to an aldehyde group, obtaining the so-called Corey aldehyde (the protected, in particular, benzoated aldehyde).

In the second reaction stage (stage B), the Corey's benzoated aldehyde (**2**) is made to condense with dimethyl-(2-oxo-4-phenylbutyl)phosphonate (**10**) 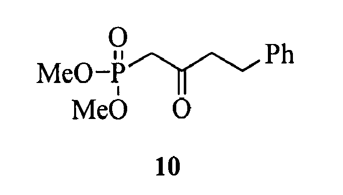 according to the method known as Horner-Wadsworth-Emmons method. The classic Wittig reaction, which takes place between one phosphorous ylide and an aldehyde or a ketone, may have several variants, particularly the one introduced by Horner, who was the first to use both phosphinoxides and phosphonates in olefination reactions with aldehydes and ketones. The special advantages offered by using phosphonates, in particular the ones with substituents in α that can stabilise the phosphonyl carbanion, were highlighted only after a study by Wadsworth and Emmons. Therefore, the olefinations taking place starting from phosphonates are called Horner-Wadsworth-Emmons reactions (HWE).

The enone (**3**) produced in stage B is subjected to a reduction reaction in the α-β-unsaturated carbonyl group to yield the corresponding alcohol function. The formation of the new chiral centre poses the question on the proper stereochemistry and thus on the need for an appropriate asymmetrical synthesis to obtain the desired product as a single isomer. Where the final product is desired as a racemate (for example, fluprostenol), then non-stereoselective reducing agents can be used, such as lithium and aluminium hydride (LiAlH₄), sodium borohydride (NaBH₄) and other metal hydrides, but in the case of latanoprost, whose configuration required at C-15 is of the (R) type (and thus is S in the intermediate (**4**)), more stereoselective reagents must be used. In the work of Resul et al., use is made, for example, of lithium tri-*sec-*butylborohydride (L-Selectride™) at a temperature of -120°C, and in these conditions the ratio obtained between the S:R configurations is of 7:3. The isomers obtained in this way must be separated by laborious and complex chromatographic methods.

Still with reference to Scheme 1, the catalytic hydrogenation of the double bond of compound (**4**) is the last reaction to be carried out to conclude the operations on the ω chain (stage D). Resul et al. performed hydrogenation in the presence of 10% Pd/C, by using ethanol as a solvent, and the hydrogen pressure was of one atmosphere; the reaction mixture was also weakly alkalinised with NaOH.

A key step for the synthesis of PGF_{2α} derivatives of the latanoprost type is the transformation of the lactone function into a lactol function, represented by stage F in Scheme 1. 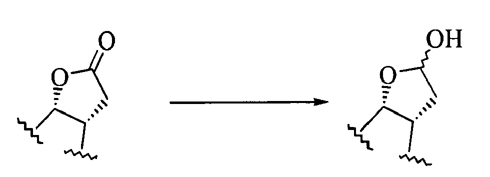 Only later, by exploiting the cyclic hemiacetal function, is the α chain bonded through a classic Wittig reaction.

Resul et al., as well as the two aforesaid patent documents WO 90/02553 and WO 93/00329, carried out the transformation of lactone into lactol by using diisobutylaluminum hydride (DIBAL-H) as a reducer. To improve the yield, Resul first carried out deprotection of the hydroxy group on the cyclopentane ring (stage E) and then the reduction of lactone at a temperature of-78°C (stage F). The overall yield is 55%.

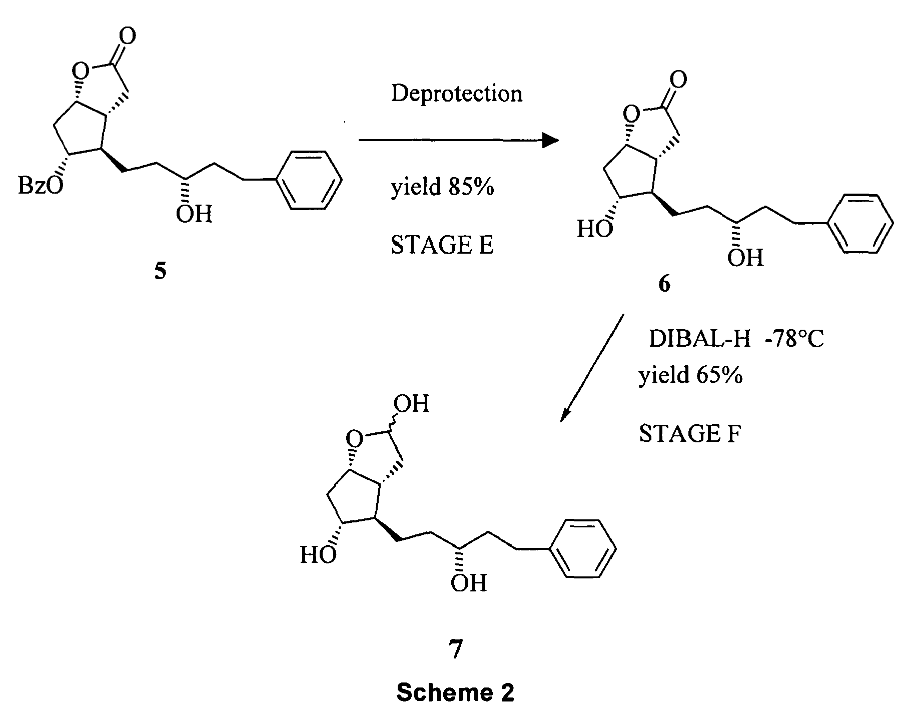

The examples of document WO 93/00329 report two further possibilities (Scheme 3): the first possibility (**a**) is the reduction of the oxo lactone group with DIBAL-H followed by deprotection, the second (**b**) is that of using an excess of DIBAL-H to also obtain the deprotection reaction simultaneously with the lactone group reduction reaction. A yield of 63% is reported for the latter process.

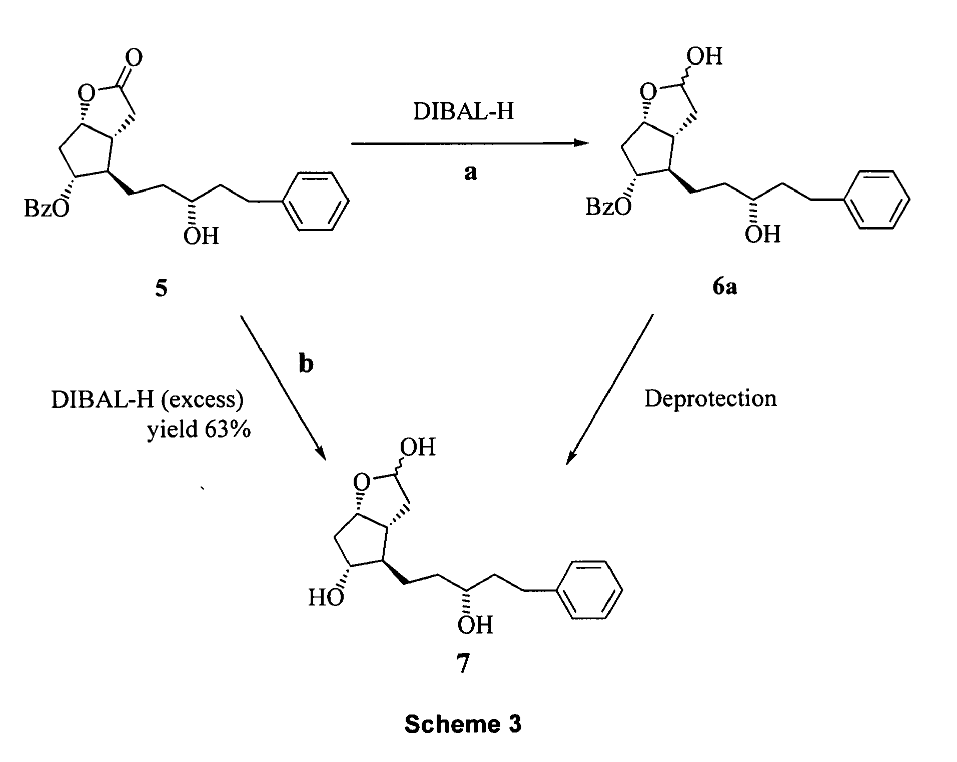

Continuing with the synthesis according to Resul, illustrated in Scheme 1, the reactivity of the cyclic hemiacetal function present in the intermediate (**7**) is used in stage G of the synthesis to bind the α chain through a classic Wittig reaction. The ylide (**26**)

Ph₃P=CH(CH₂)₃COO⁻ **26**

is generated by reacting, respectively in the molar ratio of 1:2, (4-carboxybutyl)triphenylphosphonium bromide and potassium *t*-butoxide. For the preparation of the ylide, the temperature may range from -10°C to 0°C, but is later lowered to -15°C/-5°C when the ylide is made to react with the hemiacetal function of intermediate (**7**). The molar ratio used between the cyclic hemiacetal and the ylide (**26**) is 1:4.5.

As regards extraction of the prostaglandin (**8**) from the reaction mixture, water treatment at the end of the reaction yields an alkaline solution (pH ≈ 12), which is treated with ethyl ether. This treatment aims at extracting triphenylphosphine oxide and leave in the aqueous phase the desired compound **(8)** as carboxylated compound. In the second part of the extraction process the aqueous phase is acidified up to pH = 3÷4 with citric acid, and after acidification the prostaglandin **(8)** is extracted with ethyl acetate; the organic phase is separated and then concentrated until it yields an oily pale yellow residue.

In the last reaction phase illustrated in Scheme 1, the carboxy function introduced with the α chain is esterified with isopropyl iodide ((CH₃)₂CHl) in the presence of diazabicycloundec-7-ene (DBU). The work by Resul et al. reports for the last two stages, i.e. the Wittig reaction on substrate (7) and the esterification with isopropyl iodide in the presence of DBU, an overall reaction yield of 38%.

The international patent application WO 03/008368 (Pharmacia & Upjohn), as well as the international patent application WO 01/87816, having the same owners and identical text, concern a synthesis process aimed at producing latanoprost wherein for each of the steps illustrated in Scheme 1 there are improvement solutions proposed, in order to enhance the overall yield of the process. A central object of the solution described is, however, a specific proposal for carrying out stage C of reduction of the carbonyl α-β-unsaturated group, which is carried out by using *B*-chlorodiisopinocampheylborane (Ipc₂BCl or DIP-chloride™) as a reactive for the asymmetrical synthesis. The reactive is used preferably in a ratio of 3-4 equivalents per substrate equivalent (enone (3)), at a preferred temperature ranging between -35°C and -45°C.

A further object of the solution proposed by document WO 03/008368 is a variant of the process described in Scheme 1, wherein after reduction of the aforesaid carbonyl group the product **(4)** is deprotected from the ester function in C-11 by means of a basic hydrolysis, in conditions that also open up the lactone ring of the compound **(4),** obtaining the new intermediate **(15):** 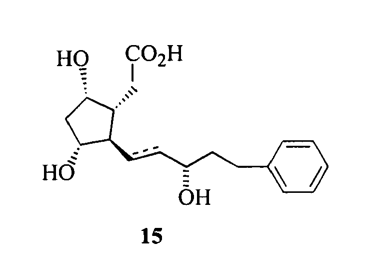 This is then lactonized once again with both the hydroxy groups free, and the latter are then both protected by reaction with ethylvinylether before undergoing the reduction reaction from lactone to lactol.

A further improved synthesis process of PGF_{2α} analogues is described in international patent application WO 02/096898 (Resolution Chemicals Ltd.). In this case, too, each of the steps of Resul's original synthesis described in Scheme 1 undergoes some modifications in order to improve the overall yields of the synthesis. Among the main solutions proposed as innovative, the document introduces, for stage A of oxidation of the alcohol group of Corey's bicyclic lactone, the use of sodium hypochlorite in the presence of a stable organic nitroxy radical, in particular a free radical 2,2,6,6-tetramethyl-1-piperidinyloxy (TEMPO). Other specific solutions concern the execution conditions of the condensation stage B according to the Horner-Wadsworth-Emmons method, for which the use of weak organic bases is proposed, such as 1,8-diazabicyclo[5.4.0]undec-7-ene (DBU), diisopropylethylamine and triethylamine, which create milder conditions than those used by Resul et al.. Moreover, in stage C of reduction of the α-β-unsaturated carbonyl group, oxazaborolidines are proposed as agents for enantioselective reduction, and various other specific solutions are illustrated also for the subsequent stages of the process. In this case, too, as in all the previous literature, the reduction of the lactone ring to lactol is carried out with the reducing agent diisobutylaluminum hydride (DIBAL-H).

The latanoprost synthesis process described in international patent application WO 01/55101 (Finetech Ltd.) focuses on the step corresponding to stages E and F of Scheme 1 in order to improve the yields of the reduction phase of lactone to lactol. In this case, the lactone intermediate (4) (with the steric configuration desired) is not deprotected in C-10, but is protected also in the hydroxy group in C-15, and is then subjected to hydrogenation of the double bond, and it is the derivative with both hydroxy groups protected that is subjected to reduction with DIBAL-H.

With reference to the synthesis phase of the hemiacetal intermediate (7) (lactol), it must be noted that all the known literature on latanoprost synthesis envisages the transformation of lactone into lactol by using DIBAL-H as a reducing agent. The synthetic procedures adopted to improve the yield of this stage are many. As already noted, in the works of Resul et al. first the deprotection of the hydroxy group on the cyclopentane ring (stage E) is carried out and then the reduction of the lactone at a temperature of -78°C (stage F), with an overall yield of 55%. The reductions carried out using DIBAL-H are strongly exothermic reactions and this is a serious difficulty if it is considered that these reactions must be carried out at temperatures around -80°C. This difficulty becomes a greater problem when it is desired to pass to synthesis on a large scale. Indeed, when the reduction reaction with DIBAL-H is carried out at temperatures higher than those normally used to reduce the lactone group, unwanted products are obtained, with a consequent decrease in yield.

According to the procedure proposed by the latter cited document, WO 01/55101, it is possible to obtain a reduction of the lactone group with diisobutylaluminum hydride at temperatures ranging between -20°C and +20°C - temperatures that are better tolerated from an industrial standpoint, protecting the hydroxy group in C-15 with tetrahydropyranyl (THP), or with other equivalent protector groups. The reaction scheme for the step concerned is as follows:

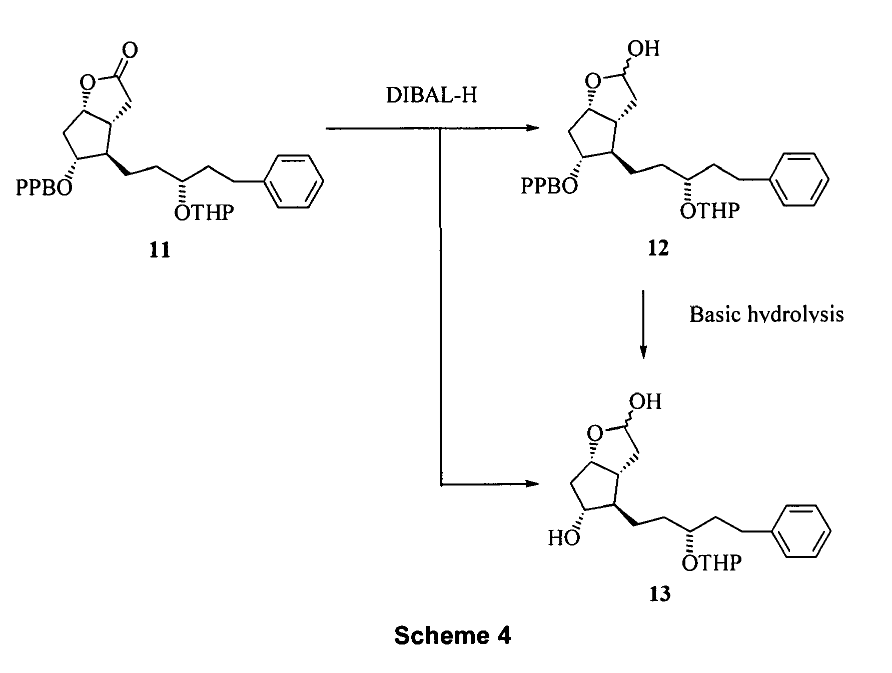

In this case, besides reducing, the DIBAL-H performs a partial deprotection which will be then completed through basic hydrolysis with potassium carbonate. After chromatographic separation on silica gel, the tetrahydropyranyl-derivate (**13**) is obtained pure with a yield of 88%. The product (**13**) then undergoes the Wittig reaction to introduce the α chain and is subsequently deprotected. 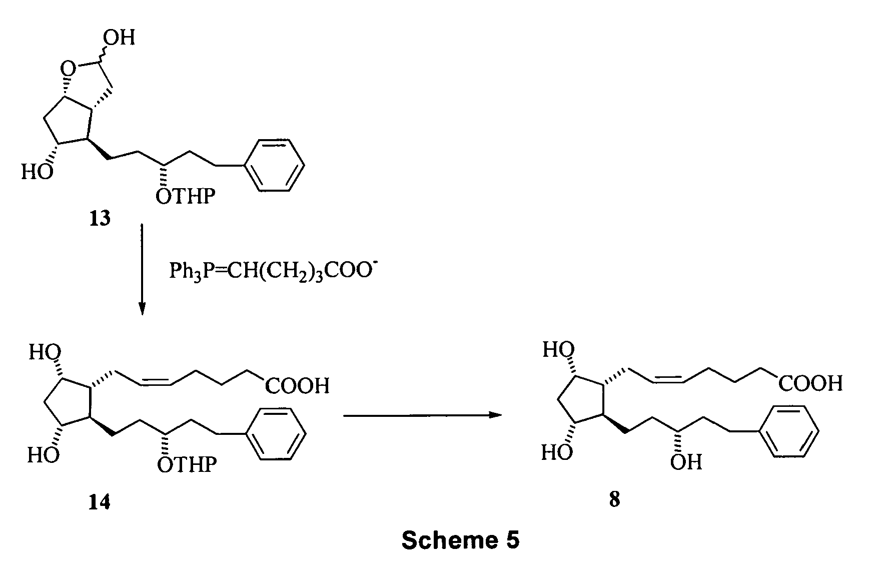 According to the aforesaid document, a further advantage of this procedure is that of obtaining intermediate compounds (of the type as **11)** which are easy to crystallise, since they are protected by hydroxy groups.

A similar synthesis strategy with protection and deprotection is used also in the already cited document WO 03/008368 (Pharmacia & Upjohn), of which Scheme 6 below shows the entire synthesis process. As already noted, in this case the unsaturated intermediate **(4)** or saturated intermediate **(5)** is deprotected from the ester function in hydrolysis conditions such to open also the lactone ring, to obtain the intermediate **(15).** From this it is then possible to continue the synthesis until latanoprost and its unsaturated analogue is obtained through lactonization, protection of the hydroxy groups by reacting with ethylvinylether and reduction with DIBAL-H of the protected intermediate (**17**). At this point the Wittig reaction takes place to introduce the chain in α and then the deprotection reaction with concentrated phosphoric acid.

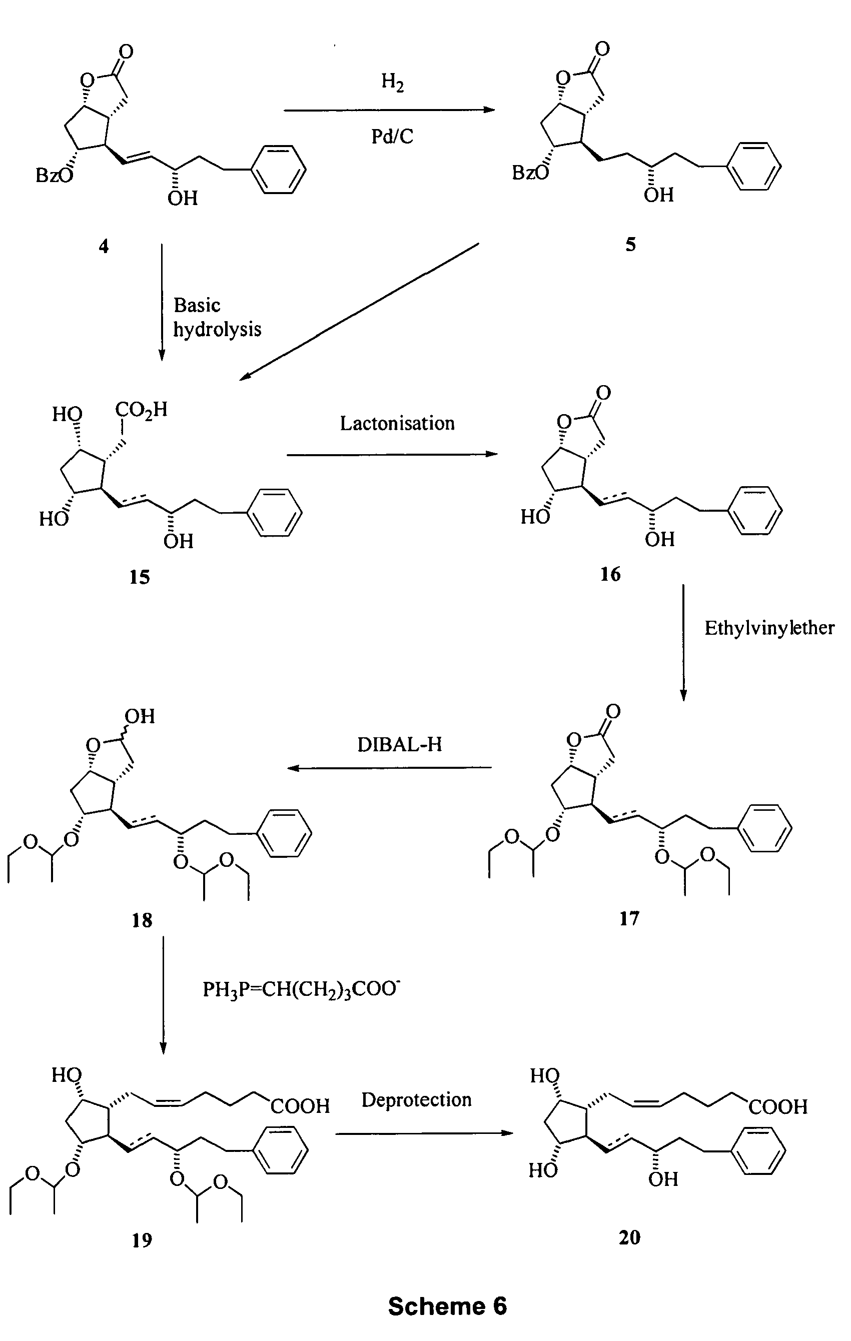

Finally, also the reduction with diisobutylaluminum hydride of the oxo lactone group described in document WO 02/096898 (Resolution Chemicals, already cited) is preceded by an hydroxy groups protection reaction, which is carried out by means of trialkylchlorosilane, according to schemes that do not differ much from the ones described above.

In view of the above, it appears that for the second part of the synthesis of PGF_{2α} derivatives of the latanoprost type, consisting of introducing the α chain in the molecule, the literature reports as an elective method the preventive reduction with DIBAL-H, followed by the introduction of the desired chain through a Wittig reaction. However, besides being pyrophoric and sensitive to air, DIBAL-H poses other problems which make large scale synthesis difficult, in particular for the low temperature at which it must be used and the simultaneous exothermicity of the reaction. To improve reaction conditions in order to pass more easily to large scale synthesis, the prior art illustrated above avoids direct reduction of the deprotected lactone intermediate **(5)** with DIBAL-H, and uses synthesis strategies making extensive use of protection and deprotection of hydroxy groups. Through these procedures the general reaction conditions are improved, but bearing in mind the losses incurred in the protection and deprotection stages, the overall yields of the reduction product **(7)** or of its protected derivatives are not considerably improved with respect to the direct reduction of the intermediate **(5).**

Therefore, an object of the present invention is to provide a method for the synthesis of latanoprost and, in general, of prostaglandin derivatives of the F type, which avoids the reduction with DIBAL-H to convert the lactone group of the compound in preparation into the corresponding lactol, avoiding the use of reaction temperatures that are too low and difficult to use.

To this end, according to the present invention, the possibility has been considered to use a kind of reaction known in the literature for the reduction of lactones into lactols, catalysed by titanocene, which performs a hydroxylation of the lactone (Verdaguer, X., et al., J. Org. Chem. 62, (1997), 8522), which does not appear to have ever been used for the reduction of lactone intermediates in the prostaglandin derivative synthesis process. This synthesis, which is carried out at room temperature, uses inexpensive reagents that are stable in air, titanocene difluoride and polymethylhydroxysilane (PMHS), and has been applied with good yields to a discrete variety of substrates containing 5- or 6-membered lactone rings.

The mechanism the reaction is based on is described in the following schemes. The titanocene difluoride **(21)** (Cp₂TiF₂, where each Cp represents a cyclopentane ring coordinated by the titanium atom), in the presence of silane (R₃SiH) forms the active species (**22**), a hydride of trivalent titanium (Scheme 7).

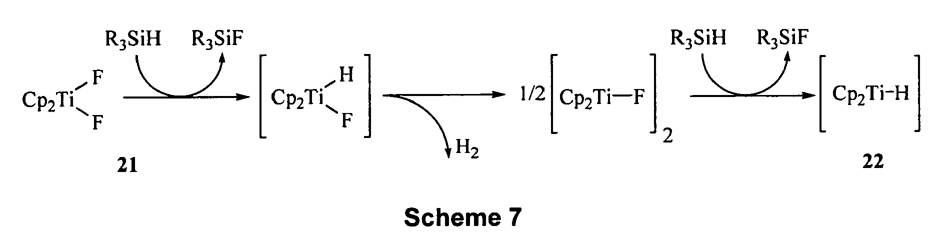

The active species **(22)** binds to the lactone carbonyl to form a titanium alkoxide **(23);** the latter reacts with the silane to form a silylated lactol **(24)** and regenerating the titanium hydride (Scheme 8). The lactol is then freed from the silyl group by basic treatment during the workup.

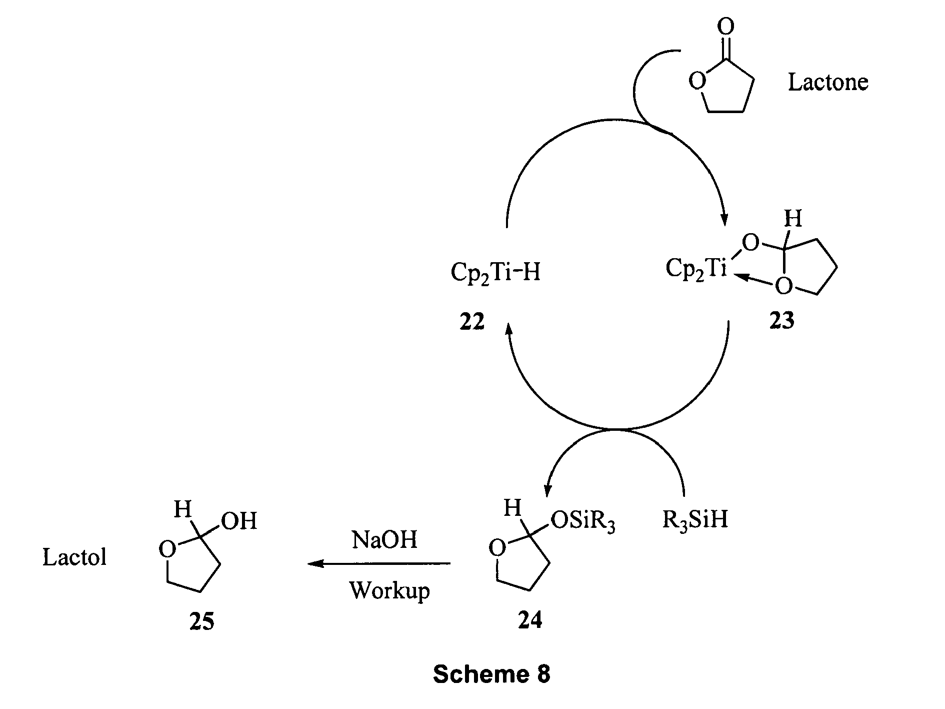

The application of the aforesaid reduction to the lactone intermediate **(5)** (see Scheme 1) in the synthesis of prostaglandin derivatives is not suggested by the prior art, most likely due to the presence - in the substrate **(5)** to be reduced - of a second ester function. The latter, in fact, being also subject to hydrosilylation, may create bridging bonds between molecules of the siloxane polymer with the consequent gel formation. In these cases, the authors of the cited study suggest using phenylsilane instead of PMHS, in this way renouncing the use of an inexpensive and safe reactive like PMHS.

According to the invention, it has been found that by first proceeding to the activation of the catalyst in the experimental conditions described by the cited reference and only later adding a solvent in small doses, it is possible to avoid the formation of a rubbery gel, and it has been possible to protract the reaction for 5 hours. Hence, the reduction of lactone (**5**) with PMHS in the presence of titanocene difluoride according to the aforesaid procedure (Scheme 9) yielded the intermediate (**7**), after chromatographic purification, with a yield of 70%.

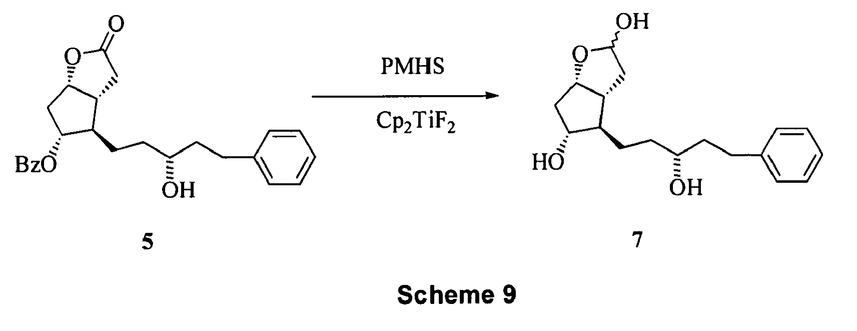

After "clean up" of the reaction mixture, it was also possible to crystallise the lactol (**7**); in this case 86% of the product was recovered, with a final yield of 60%. The crystallised product was subjected to ¹H, ¹³C, COSY and APT spectroscopic analysis. An accurate study of the spectra enabled attributing the structure of the hemiacetal (**7**) to the crystalline compound.

Therefore, a specific object of the present invention is a process for the synthesis of a prostaglandin F derivative wherein to a prostaglandin cyclopentane ring modified with a lactone function, an α chain is connected in a first phase, and an ω chain is connected in a second phase, upon transforming the said lactone function into lactol, characterised in that the transformation of the lactone intermediate into lactol is obtained by treating the said lactone intermediate with a silane in the presence of a titanocene.

Preferably, the said silane is polymethylhydrosiloxane (PMHS) and the said titanocene is chosen from among titanocene difluoride and titanocene diphenoxide.

According to the optimal procedure proposed by the present invention, the treatment of the lactone intermediate with PMHS in the presence of a titanocene is carried out by first bringing the silane into contact with the titanocene in a suitable organic solvent, preferably tetrahydrofurane, and only later, after the formation of the catalytically active species from titanocene, by adding the lactone intermediate to the reaction mixture. Still according to the preferred procedure, the cited organic solvent is added to the reaction mixture in small doses in two or more steps, in order to reduce the formation of a rubbery gel in the reaction mixture and to protract the reaction itself as much as possible.

The synthesis method proposed is applicable in particular to the synthesis of type F_{2α} prostaglandin derivatives and is particularly advantageous in the case where the PGF_{2α} derivative is latanoprost.

In this case, the cited transformation of the lactone intermediate into lactol takes place according to the following scheme: 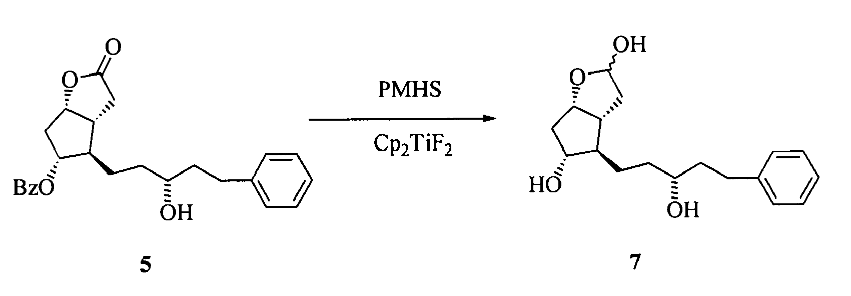 wherein Bz is a benzoyl protective group, PMHS is polymethylhydrosiloxane and Cp₂TiF₂ is titanocene fluoride. Instead of the benzoyl group, another protecting group may be envisaged - in particular, a silylether group.

Whatever the PGF to which the process according to the present invention is applied, on the basis of a preferred synthesis solution, the commercially available starting compound is Corey's benzoated aldehyde (compound (**2**) in Scheme 1). Starting from the said compound, in the application of the synthesis method proposed to latanoprost preparation, the Corey's benzoated aldehyde (**2**) is subjected to the following reaction stage: 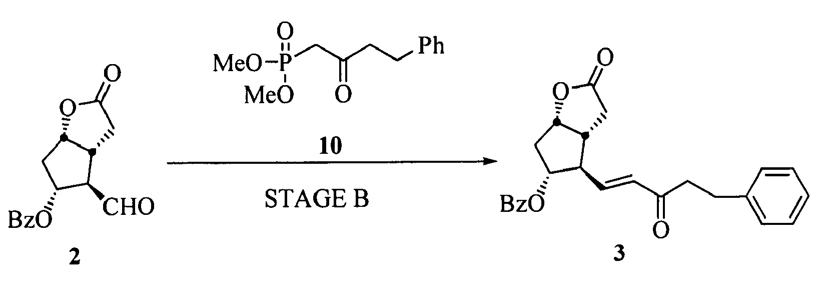 consisting of the introduction of the α chain by condensation through the Hor-Horner-Wadsworth-Emmons reaction with dimethyl-(2-oxo-4-phenylbutyl)phosphonate **(10).** Preferably, the stage B reaction is carried out with a weak organic base, such as diisopropylethylamine or triethylamine, in the presence of lithium or magnesium salts. By appropriately coordinating with the oxygen atoms of the phosphonate, these salts enable the formation of the phosphonyl carbanion, thus enabling milder reaction conditions than those originally adopted by Resul, who used a strong base for the same purpose.

According to the preferred synthesis process of the present invention, the enone (**3**) obtained from stage B is subjected to the following reaction stage: 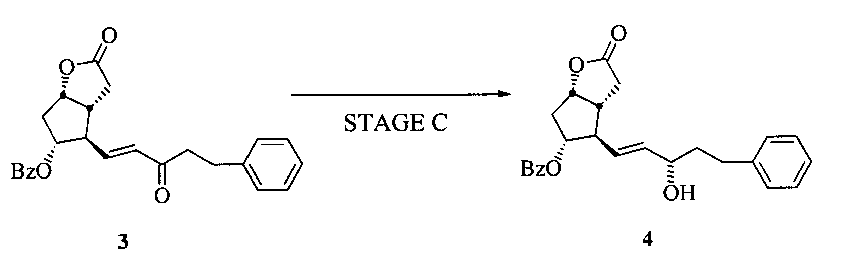 consisting of the stereoselective reduction of the α-β-unsaturated carbonyl group to yield the corresponding alcohol function.

As already noted, for this step it is preferred to use a stereoselective reducing agent, in particular *B*-chlorodiisopinocampheylborane (Icp₂BCl or DIP-Chloride™), in temperature conditions between -20°C and -45°C. In particular, the said reduction is carried out in the presence of hexane with a quantity of *B*- chlorodiisopinocampheylborane 4-6 times more than the stoichiometric quantity, and preferably 5 times more, and at a temperature ranging between -30°C and -33°C. In these conditions, yields of 95% are obtained, with an enantiomeric excess of 94%.

Still following the preferred synthesis process for latanoprost, the alcohol (**4**) obtained from stage C is subjected to the following reaction stage: 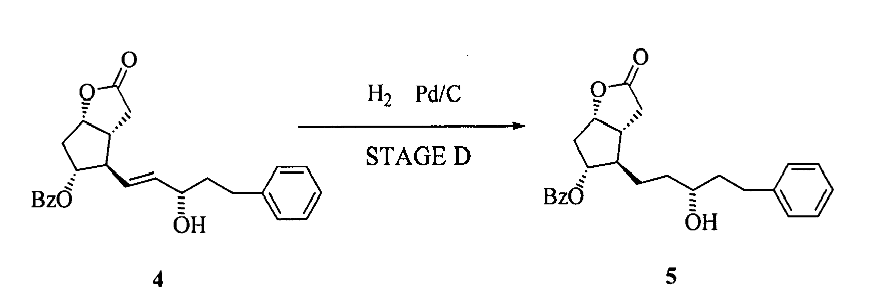 consisting of a catalytic hydrogenation of the double bond in the presence of palladium on carbon. This reaction can take place in solvents such as ethanol, tetrahydrofurane or ethyl acetate in the presence of Pt/C or Pd/C and at hydrogen pressures ranging between 5 psi and 150 psi, and is preferably carried out, according to the present invention, in ethyl acetate at a pressure of 45 psi in the presence of 5% Pd/C, and also in the presence of triethylamine at about 0.3% v/v.

The lactone intermediate **(5)** obtained from stage D is then subjected to a stage E-F of transformation of the lactone function into lactol as previously described, according to the main feature of the present invention, and subsequently the lactol **(7)** obtained from stage E-F is subjected to the following reaction stage: 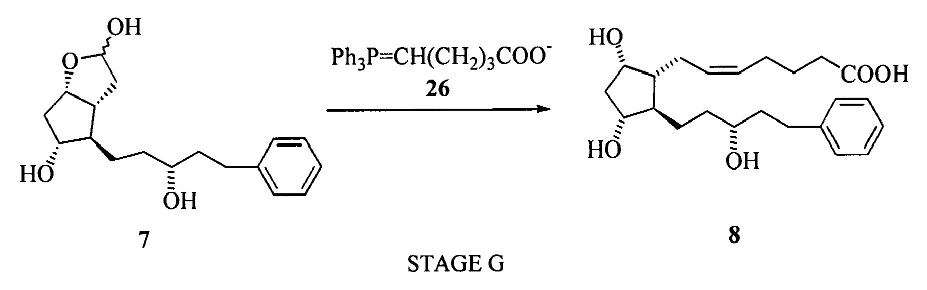 consisting of introducing the ω chain by condensation via a Wittig reaction with an ylide (**26**). In carrying out the process according to the present invention, the chromatographic check of the mixture at the end of the reaction by TLC enabled observing the total disappearance of lactol **(7)** (100% conversion) and the formation of a single product, besides the secondary products resulting from ylide (**26**), triphenylphosphinoxide and (4-carboxyybutyl)diphenylphosphin. At the end of the reaction, the excess of the reactive **(26)** is hydrolysed with water and this operation creates further quantities of triphenylphosphinoxide and di (4-carboxyibutyl)diphenylphosphin.

As regards the extraction of the prostaglandin (**8**) obtained, the treatment with water at the end of the reaction provides an alkaline solution (pH ≈ 12) which is treated with an organic solvent in order to extract the triphenylphosphinoxide and leave in the aqueous phase the desired compound **(8)** as carboxylate. According to the present invention, the prostaglandin **(8)** is extracted from the reaction mixture of stage G by treatment with ethyl ether, and then the aqueous phase is acidified up to pH 3-4 with 10% citric acid in the presence of ethyl ether. During this operation there is the formation of small quantities of precipitate that is filtered and discarded. Subsequently, the ether phase is separated and concentrated up to obtaining a second precipitate, which is also separated by filtering. The filtrate is concentrated up to obtaining an oily pale yellow product.

The crystalline precipitates obtained in the acidification phase and in the concentration of the solvent, as well as the oily product, were subjected to chromatographic analysis by TLC. The crystals turned out to be a single compound, (4-carboxybutyl)diphenylphosphin, while thin layer chromatography of the oily final product showed - besides the compound of interest (**8**) - appreciable quantities of triphenylphosphinoxide and of (4-carboxyibutyl)diphenylphosphin.

Still according to a preferred embodiment of the present invention, the prostaglandin **(8)** obtained from stage G is subjected to the following reaction stage: 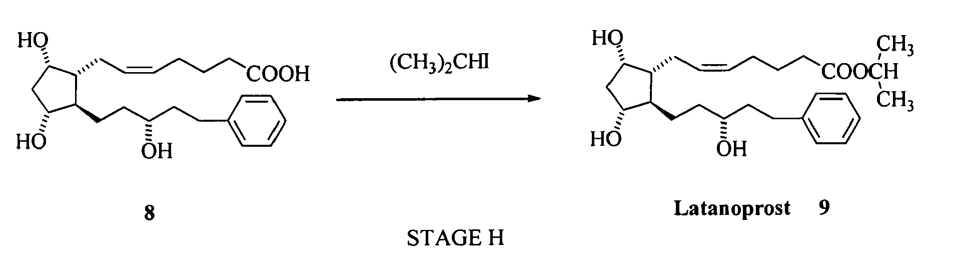 consisting of its esterification with isopropyl iodide (CH₃)₂CHl. This reaction may be carried out in the presence of diazabicycloundec-7-ene (DBU) or caesium carbonate, the second reactive being the one preferred according to the present invention. Preferably, the process employs isopropyl hydride, caesium carbonate and the prostaglandin **(8)** in molar ratios of 2:2:1, with DMF (dimethylformamide) as a reaction solvent and a temperature around 45°C.

The reaction mixture may then be extracted with MTBE-water and the organic phase separated and then concentrated up to providing an oily residue. By silica gel column chromatography, a chromatographically pure fraction was isolated from the previous reaction product and was subjected to spectrosopic analysis, confirming with certainty that the isolated product has the structure of latanoprost. The chromatographic purification also provided another fraction containing latanoprost in the presence of 20% of triphenylphosphinoxide.

A specific embodiment of the process according to the present invention, applied to the synthesis of latanoprost according to the general lines of Scheme 1, but with the innovative modifications proposed on the basis of the present invention, is described for exemplification purposes below. As already noted, since the Corey's benzoated aldehyde (2) is commercially available at a price just a little higher than the one of the corresponding alcohol (1), the synthesis shown starts from the intermediate (2), avoiding stage A of oxidation of the alcohol group of the Corey lactone.

### EXAMPLE OF SYNTHESIS

### Materials and Methods

The ¹H and ¹³C NMR spectra were recorded on a Varian Unity Inova spectrometer at 500 and 125.7 MHz, respectively.

The dimethyl-(2-oxo-4-phenylbutyl)phosphonate was purchased from Acros Organics. The Corey's benzoated aldehyde, (-)-Dip-Chloride, PMHS, lithium chloride, caesium carbonate and 2-iodopropane were purchased from Sigma-Aldrich-Fluka. THF, triethylamine, MTBE, acetonitrile and *n*-hexane were purchased from Merck. Tetrahydrofurane was anhydrified on metallic sodium in the presence of benzophenone and triethylamine was anhydrified on CaH₂ and preserved on anhydrous K₂CO₃.

The *n*-heptane, isopropanol, ethyl ether and ethyl acetate were purchased from Lab-Scan.

Titanocene difluoride was synthesised in agreement with what is described in the literature (Druce, P. M., et al., J. Chem. Soc. A, (1969), 2106). To 1 g of titanocene dichloride dissolved in 32 ml of H₂O was added 0.4 g of NaF and the suspension was left under magnetic stirring for 1 h. The titanocene difluoride was separated from the solution by filtering and then crystallised by THF.

### STAGE B

In a 100 ml flaskl containing 0.62 g of anhydrous lithium chloride, the following were added: 20.5 ml of anhydrous tetrahydrofurane (THF), 2.4 g of dimethyl-(2-oxo-4-phenylbutyl)phosphonate (85% purity) and 1 ml of anhydrous triethylamine. After adding ammine, a milky suspension was formed which was placed in an alcohol bath at a temperature of -10°C. To this was added, in the course of 3 h, the Corey's benzoated aldehyde (2 g in 18 ml of THF), and the mixture was left to react for 10 h. Hence, it was placed at a temperature of -18°C for another 12 h.

After this time, 15 ml of methyl *tert*-butyl ether (MTBE) was added and, over 1 hour, the temperature was increased to 20°C.

The mixture was transferred to a funnel separator and was extracted with 15 ml of a 38% sodium bisulphate solution. The aqueous phase was discarded and the organic phase was again extracted with a saturated sodium bicarbonate solution.

The organic phase was collected in a 100 ml ball and concentrated to a volume of about 6 ml; 25 ml of ethyl acetate was then added and the volume was again reduced until a white precipitate was formed. The precipitate was re-dissolved under heating in the minimum quantity of ethyl acetate and, still in hot conditions, about 10 ml of MTBE was added a little at a time until a slight clouding was obtained. The solution was then left to cool to reach room temperature and then taken to -18°C for 2 h. After discarding the crystallisation waters by decanting, the precipitate was washed with cold MTBE.

The product obtained, corresponding to the desired enone (3), was then dried with a weak nitrogen flow (1.85 g yield 65%). ¹H NMR (CDCl₃, 500 MHz) was in agreement with the literature.

### STAGE C

In a 100 ml flask equipped with a perforatable cap, 1.8 g of enone (**3**) was dissolved in 18 ml of anhydrous THF and placed in an alcohol bath at a temperature ranging between -30 and -33°C. To this mixture was added, drop by drop using a syringe, 7.8 ml of a solution of (-)-Dip-chloride 2M in hexane.

After 18 h of reaction, 2.5 ml of acetone was added. The reaction mixture was then heated over 30 min up to a temperature of 20°C and left under stirring for another 2 h.

To the contents of the ball was added 18 ml of MTBE, and the whole mixture was transferred to a funnel separator. The mixture was then extracted with 27 ml of a saturated solution of NaHCO₃.

The organic phase was again extracted with 18 ml of H₂O; the aqueous phase was discarded and the organic phase was concentrated to a small volume.

Then 54 ml of MTBE was added, the solvent was evaporated again, 18 ml of CH₃CN was added, and the mixture was reduced to a small volume.

The concentrated solution was taken again with 27 ml of CH₃CN, transferred to a funnel separator, and extracted 3 times with 18 ml of n-heptane. The acetonitrile phase was collected inside a 100 ml ball and evaporated under vacuum inside a centrifugal separator. The product obtained was purified by silica gel chromatography, by using an ethyl acetate gradient in hexane. 1.58 g of the pure product **(4)** was obtained, with a yield of 88%. ¹H NMR (CDCl₃, 500 MHz) was in agreement with the literature.

### STAGE D

1.58 g of the product (**4**) was dissolved in 75 ml of ethyl acetate; then 263 mg of 5% Pd/C and 240 µl of triethylamine was added to this solution. The suspension was then subjected to a hydrogen pressure of 45 psi. After stirring for 2 h, the reaction mixture was filtered and the filtrate evaporated until an oily residue was obtained.

Silica gel chromatographic purification of the oil provided 1.51 g of the pure product **(5),** with a yield of 96%. ¹H NMR (CDCl₃, 500 MHz) was in agreement with the literature.

### STAGES E and F

### Synthesis of the intermediate (7): reduction with polymethylhydrosiloxane (PMHS)

The following were introduced, under argon, inside a twin-necked 50 ml oven anhydrified flask: 16 mg of titanocene difluoride, 4 ml of anhydrous THF and 1.3 ml (1.2 g, PM=2200÷2400) of PMHS. The mixture was stirred and placed in a water bath at a temperature of about 60°C for a few min (0.5-2 min) until a bright blue colouring appeared accompanied by bubbles. The reaction flask was placed inside an ice bath and, by using a syringe, 290 mg of lactone (**5**) - previously dissolved in 1.2 ml of anhydrous THF - was added. After adding the latter, the reaction was made to continue at room temperature.

After 15 min of reaction the mixture started to show such viscosity that it became necessary to subject it to strong magnetic stirring.

After 1 h of reaction, the gel became rubbery which, under stirring, started to break up; in this phase it was necessary to add the smallest amount of THF (6÷8 ml) until the gel reconstituted.

Stirring was continued for another 4 h. At this point the reaction mixture was exposed to air in order to deactivate the catalyst and transferred to a 100 ml flask with the help of 8 ml of THF. Then, 15 ml of NaOH 1M was carefully added under stirring. After 1 h and 30 min the mixture was transferred to a funnel separator together with 5 ml of ethyl ether. The aqueous phase was discarded and the organic phase was added with another 15 ml of ethyl ether and 10 ml of NaOH 1 M. The two phases were shaken and the aqueous phase was discarded. The organic phase was then washed with 10 ml of a saturated solution of NaCl, dried on MgSO₄ and evaporated.

The residue thus obtained yielded - after purification by silica gel chromatography - 154 mg of the product **(7)** with a yield of 70%.

Alternatively, the lactol **(7)** may be crystallised: the raw residue was dissolved in the smallest amount necessary of acetone; 3 parts of ethyl acetate were added and the volume was then reduced until the first crystals appeared. Crystallisation was completed at a temperature of 4°C in a period of 2h.

The crystals were separated by decanting (60% yield p.f. = 108-110 °C) and subjected to spectroscopy. The analysis of the NMR ¹H, ¹³C (APT), and COSY spectra, all carried out in CD₃OD, made it possible to assign the structure of triol **(7).**

¹HNMR (500 MHz, CD₃OD, TMS): the main isomer of the diastereomer mixture δ = 1.25 (m, 1 H, H-8b), 1.54-1.45 (overlapping multiplets, 2H, H-7, H-9b), 1.65-1.55 (partly overlapping multiplets, 2H, H-5b, H-9a), 1.81-1.66 (overlapping multiplets, 3H, H-11a, H-11b, H-8a), 1.92 (m, 1 H, H-2b), 2.04 (m, 1 H, H2a), 2.34-2.24 (partly overlapping multiplets, 2H, H-3, H-5a), 2.64 (m, 1H, H-12b), 2.78 (m, 1H, H-12a), 3.54 (m, 1H, H-10), 3.74 (m, 1 H, H-6), 4.54 (m, 1H, H-4), 5.53 (dd, *J*_{*1,2a*} = 1.8, *J*_{*1,2b*} = 5.0 Hz, 1 H, H-1), 7.14 (m, 1H, H-16), 7.20 (m, 2H, H-14, H-18), 7.25 (m, 2H, H-15, H-17).

¹³CNMR (125,7 MHz, CD₃OD, TMS): δ = 30.1 (C-8), 33.0 (C-12), 36.5 (C-9), 40.2 (C-11), 41.7 (C-2), 42.1 (C-5), 46.8 (C-3), 54.9 (C-7), 72.0 (C-10), 79.2 (C-6), 81.5 (C-4), 101.1 (C-1), 126.7 (C-16), 129.3 (C-14, C-18), 129.4 (C-15, C-17), 143.7 (C-13).

### STAGE G

929 mg of (4- carboxybutyl)triphenylphosphonium bromide was weighed in a 50 ml flask and suspended in 3.5 ml of anhydrous THF; to this mixture, cooled to a temperature of 0°C, was slowly added - under stirring - 520 mg of potassium *t*-butoxide previously dissolved in 4 ml of anhydrous THF.

After adding potassium *t*-butoxide, the suspension took on a redis-orange colour indicating the formation of the ylide. The latter was kept at a temperature of 0°C for 30÷40 minutes.

A solution obtained by dissolving 140 mg of lactol (**7**) in 3.5 ml of anhydrous THF was then slowly added to the phosphorous ylide at a temperature of -15°C and stirred for 4÷5 hours. The reaction may be followed by TLC by using ethyl acetate - acetic acid 97:3 v/v - as an eluent. When the whole substrate (**7**) was converted, 10 ml of water was added. The mixture was then gradually heated to room temperature in a time of 20÷30 min, transferred to a funnel separator and extracted with 10 ml of ethyl ether. The alkaline aqueous phase (pH = 12,15) was recovered and added to 10 ml of ethyl ether, and then acidified drop by drop with a solution of 10% citric acid to a pH = 3.80 (any precipitate forming during the acidification phase must be removed by filtering and discarded).

The ether phase was recovered and the aqueous phase extracted three times with 10 ml of ethyl ether.

The ether phases were collected in a 100 ml ball and then concentrated to a volume of about 15 ml. The precipitate forming during the concentration of the solvent consisted of a secondary product (4-carboxybutyl)diphenylphosphin 140 mg ), which was filtered off and discarded. The filtrate was dried on Na₂SO₄ and then evaporated until an oily residue of 219 mg weight was obtained. The raw product thus obtained was used without further purification in the next stage.

### STAGE H

In a 50 ml flask containing 456 mg of caesium carbonate, 2.9 ml of anhydrous DMF was added. To this suspension was added 219 mg of the product **(8)** previously dissolved in 4.8 ml of anhydrous DMF and then 140 µl (238 mg) of 2-iodopropane. The reaction mixture was maintained under stirring at a temperature of 45°C for a period of 5-6 h.

At the end of the reaction, the following were added consecutively: 16 ml of MTBE and 20 ml of ice water. Then everything was transferred to a funnel separator and the two phases were separated. The aqueous phase was extracted with 20 ml of MTBE. The organic phases were collected together and dried on anhydrous sodium sulphate. Once the sodium sulphate was separated by filtering, the MTBE was isolated under vacuum until a yellow oily residue weighing 235 mg was obtained. Fractionation in silica gel column, using MTBE as an eluent, yielded two fractions containing, respectively, 20 mg of latanoprost (chromatographically pure) and 75 mg of a residue containing latanoprost in the presence of 20% of triphenylphosphin oxide (the latter evaluation obtained from NMR data). Therefore, the overall yield of the last two reaction stages was 42%.

An accurate study of the NMR ¹H (CDCl₃, Figure 5), ¹H (C₅D₅N), ¹³C (APT, CDCl₃), COSY (CDCl₃), GHSQC (CDCl₃) and NOESY1 D (C₅D₅N) spectra enabled attributing with certainty the latanoprost structure to the isolated product.

¹HNMR (500 MHz, CDCl₃, TMS): δ = 1.23 (d, J = 6.3 Hz, 6H, CH(CH₃)₂), 1.34 (m, 1 H, H-13b), 1.40 (m, 1H. H-8), 1.52 (m, 1 H, H-13a), 1.62 (m, 3H, H-14, OH-15), 1.73-1.65 (partly overlapping multiplets, 3H, H-12, H-3), 1.82-1.75 (partly overlapping multiplets, 2H, H-16), 1.87 (m, 2H, H-10), 2.13 (m, 2H, H-4), 2.22 (m, 1 H, H-7b), 2.28 (t, J = 7.3 Hz, 2H, H-2), 2.32 (overlapping multiplets, 2H, H-7a, OH-9) 2.54 (d, J = 6.5 Hz, 1 H, OH-11), 2.68 (m, 1 H, H-17b), 2.80 (m, 1 H, H-17a), 3.67 (m, 1 H, H-15) 3.95 (m, 1 H, H-11), 4.18 (m, 1 H, H-9), 5.00 (heptuplet, J = 6.3 Hz, 1 H, CH(CH₃)₂), 5.40 (ddd, *J*_{*5,6*} = 10.3, *J*_{*5,4a*} = 7.1, *J*_{*5,4b*} = 7.2 Hz, 1H, H-5,), 5.46 (ddd, *J*_{6,5} = 10.3, *J*_{*6,7a*}*,* = 7.1, *J*_{*6,7b*} *=* 7.4 Hz, 1H, H-6,), 7.18 (m, 1H, H-21), 7.21 (m, 2H, H-19, H-23), 7.29 (m, 2H, H-20, H-22).

¹³CNMR (125,7 MHz, CDCl₃, TMS): δ =21.8 (CH(C*H*₃)₂), 24.9 (C-3), 26.6 (C-4), 26.9 (C-7), 29.7 (C-13), 32.1 (C-17), 34.0 (C-2), 35.8 (C-14), 39.1 (C-16), 42.5 (C-10), 51.9 (C-8), 53.0 (C-12), 67.6 (C*H*(CH₃)₂), 71.3 (C-15), 74.8 (C-9), 78.8 (C-11), 125.8 (C-21), 128.4 (C-19, C-20, C-22, C-23), 129.3 (C-6), 129.6 (C-5), 142.1 (C-18), 173.5 (C-1).

As may be seen from the foregoing, the latanoprost synthesis may ideally be subdivided into two parts: in the first part, the phenyl-substituted ω chain is introduced on the cyclopentane unit characteristic of prostaglandins, while in the second part, the α chain is introduced. For the first part, which develops in three stages, methods taken from the ones reported in the literature were used, but modifying them slightly in some cases in order to facilitate the synthesis process and to optimise the yields. The overall yield of this part of the process was 55%.

As regards the second part, that is, the introduction of the α chain, the alternative synthesis process proposed according to the present invention - consisting of reducing the lactone to lactol with PMHS in the presence of titanocene difluoride, contrary to what happens with DIBAL-H - offered the chance to carry out the reaction at room temperature with yields of 70%, without needing to protect the hydroxy groups, and thus resulting in a clearly more advantageous method.

Unlike DIBAL-H, PMHS - which accounts for 5% of the industrial production of silicone - has the advantage of being stable in air and water, and is also soluble in many organic solvents. The use of PMHS in the presence of titanocenes for transforming the lactone group into a lactol group thus provides the possibility of an alternative and advantageous reduction process both for latanoprost synthesis and for other similar prostaglandins.

The last two reaction stages, in which the lactol (7) obtained according to the present invention was converted into latanoprost (9), yielded the desired compound with an overall yield of 42%. The only control for this result is provided by the study (cited in the introduction) by Resul et al., which reports an overall yield of 38% for these two stages, using the consolidated synthesis process of the prior art.

The present invention has been disclosed with particular reference to some specific embodiments thereof, but it should be understood that modifications and changes may be made by the persons skilled in the art without departing from the scope of the invention as defined in the appended claims.

## Claims

1. A process for the synthesis of a prostaglandin F derivative wherein to a prostaglandin cyclopentane ring modified with a lactone function, an α chain is connected in a first phase, and an ω chain is connected in a second phase, upon transforming the said lactone function into lactol, **characterised in that** the transformation of the lactone intermediate into lactol is obtained by treating the said lactone intermediate with a silane in the presence of a titanocene.

2. A process according to claim 1, wherein the said silane is polymethylhydrosiloxane (PMHS).

3. A process according to claim 2, wherein the said treatment of the lactone intermediate with PMHS in the presence of a titanocene is carried out by first putting the said PMHS into contact with the said titanocene in a suitable organic solvent and then adding the said lactone intermediate to the reaction mixture.

4. A process according to claim 3, wherein the said organic solvent is added to the reaction mixture in two or more steps.

5. A process according to any one of claims 1-4, wherein the said titanocene is chosen from among titanocene difluoride and titanocene diphenoxide.

6. A process according to any one of claims 1-5, wherein the said prostaglandin F derivative is a PGF_{2α} derivative.

7. A process according to claim 6, wherein the said PGF_{2α} derivative is latanoprost.

8. A process according to claim 7, wherein the said transformation of the lactone intermediate into lactol takes place according to the following scheme: 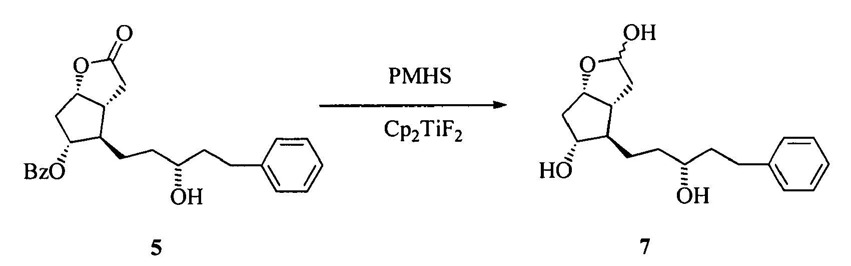 wherein Bz is a benzoyl protective group, PMHS is polymethylhydrosiloxane and Cp₂TiF₂ is titanocene fluoride.

9. A process according to claims 1 or 2, wherein the said cyclopentane ring modified with a starting lactone function is Corey's benzoated aldehyde.

10. A process according to claim 9, wherein the said prostaglandin F derivative is latanoprost and wherein the said Corey's benzoated aldehyde (2) is subjected to the following reaction stage: 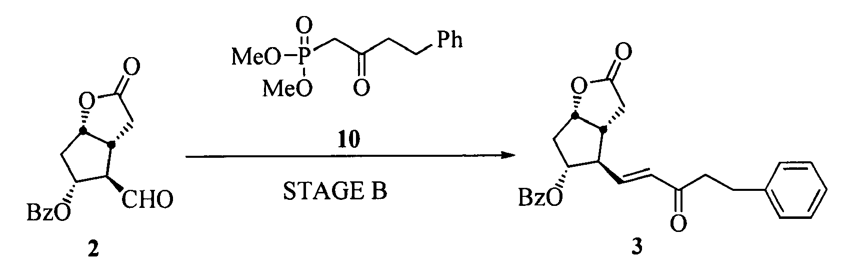 consisting of the introduction of the α chain by condensation through the Horner-Wadsworth-Emmons reaction with dimethyl-(2-oxo-4-phenylbutyl)phosphonate **(10).**

11. A process according to claim 10, wherein the said stage B reaction is carried out with a weak organic base in the presence of lithium or magnesium salts.

12. A process according to claim 9 or 10, wherein the enone (3) obtained from stage B is subjected to the following reaction stage: 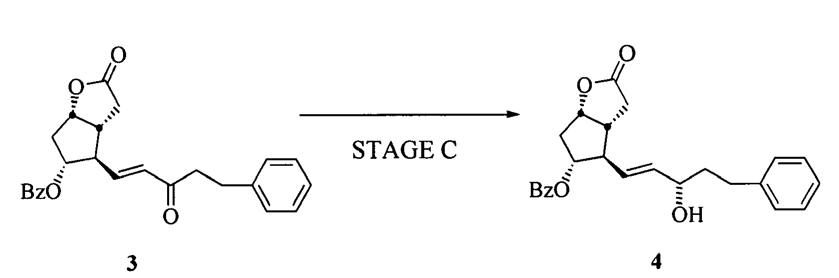 consisting of a stereoselective reduction of the α-β-unsaturated carbonyl group to yield the corresponding alcohol function.

13. A process according to claim 12, wherein the said stereoselective reduction is carried out in the presence of *B*-chlorodiisopinocampheylborane at temperatures ranging between -20°C and -45°C.

14. A process according to claim 13, wherein the said stereoselective reduction is carried out in the presence of hexane with a quantity of *B*-chlorodiisopinocampheylborane 4-6 times more than the stoichiometric quantity.

15. A process according to any one of claims 12-14, wherein the alcohol (4) obtained from stage C is subjected to the following reaction stage: 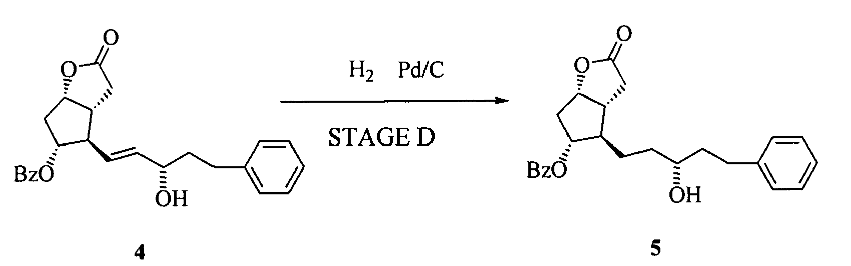 consisting of a catalytic hydrogenation of the double bond in the presence of palladium on carbon.

16. A process according to claim 15, wherein the lactone intermediate (5) obtained from stage D is subjected to a stage E-F of transformation of the lactone function into lactol, as reported in claim 8.

17. A process according to claim 15, wherein the lactol (7) obtained from stage E-F is subjected to the following reaction stage: 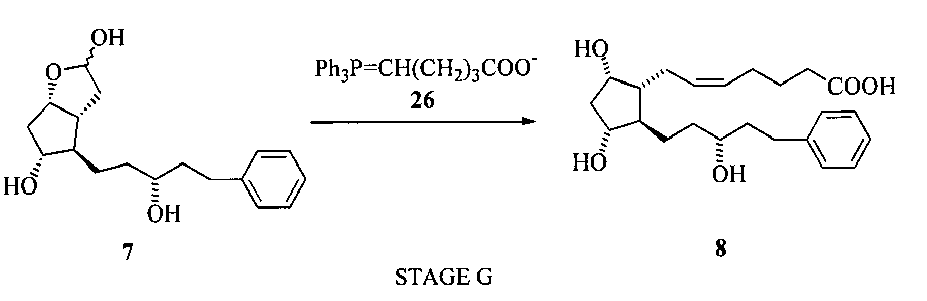 consisting of introducing the ω chain by condensation via a Wittig reaction with an ylide (26).

18. A process according to claim 17, wherein the prostaglandin (8) is extracted from the reaction mixture of stage G by treatment with ethyl ether; the aqueous phase is acidified with citric acid in the presence of ethyl ether and the ether phase is separated and concentrated.

19. A process according to claim 17 or 18, wherein the prostaglandin (8) obtained from stage G is subjected to the following reaction stage: 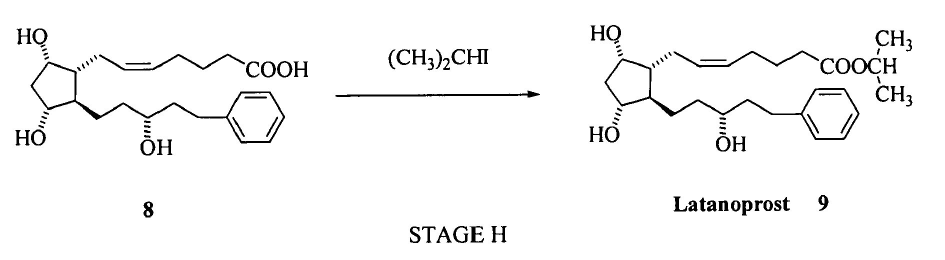 consisting of esterification of the prostaglandin (8) with isopropyl iodide (CH₃)₂CHl.

20. A process according to claim 19, wherein the said esterification is carried out in the presence of caesium carbonate.
